# EUROPÄISCHE PATENTSCHRIFT

(11) **EP 1 801 107 B1**
(45) Veröffentlichungstag und Bekanntmachung des Hinweises auf die Patenterteilung: **15.07.2009**
(21) Anmeldenummer: 06118403.2
(22) Anmeldetag: 29.10.2004
(51) Int. Cl.: C07D 263/10

(54) **Verfahren zur Herstellung von Phenylenbisoxazolinen**
Process for the preparation of phenylenebisisoxazoles
Procédé pour la préparation des phenylènebisisoxazoles

(30) Priorität: 23.12.2003 DE 10360757
(43) Veröffentlichungstag der Anmeldung: 27.06.2007
(62) Teilanmeldung aus: 04105404.0
(73) Patentinhaber: Evonik Degussa GmbH, 45128 Essen (DE)
(72) Erfinder: Meyer, Oliver, 48145, Münster (DE); Neumann, Manfred, 45770, Marl (DE); Kirchhoff, Jochen, 59348, Lüdinghausen (DE)

(56) Entgegenhaltungen:
- EP-A- 0 478 151
- DE-A- 2 158 615
- US-A- 4 574 157
- PATENT ABSTRACTS OF JAPAN Bd. 017, Nr. 513, 16. September 1993 (1993-09-16) & JP 05 140129 A (KAO CORPORATION), 8. Juni 1993 (1993-06-08)

## Beschreibung

Die vorliegende Erfindung betrifft ein Verfahren zur Herstellung von Phenylenbisoxazolinen (I), insbesondere von 2,2'-(1,4-Phenylen)bis-[4,5-dihydro-1,3-oxazol] und 2,2'-(1,3-Phenylen)bis-[4,5-dihydro-1,3-oxazol], durch Umsetzung von Terephthalodinitril bzw. Isophthalodinitril (II) mit 1,2-Aminoalkoholen (III) nach der allgemeinen Gleichung 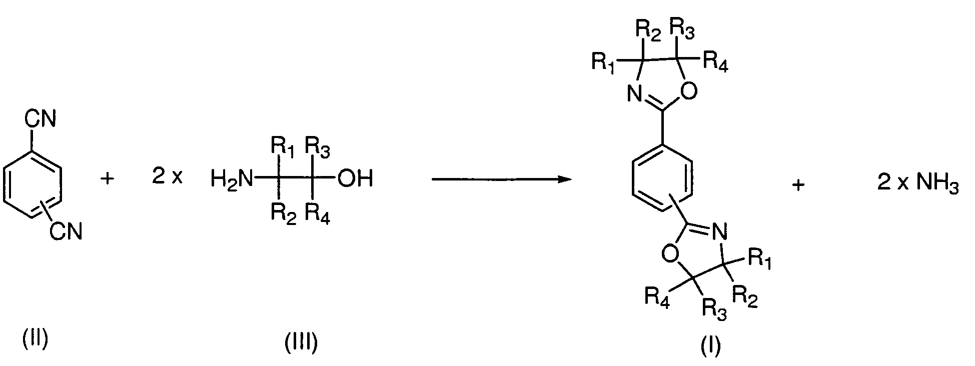 wobei R₁,R₂,R₃,R₄ gleich oder unabhängig voneinander Wasserstoff, Methyl, Ethyl, n-Propyl oder iso-Propyl bedeuten können.

Die durch das erfindungsgemäße Verfahren zugänglichen Phenylenbisoxazoline können u.a. als Kettenverlängerer oder Stabilisator in Polymeren, insbesondere in Polyestern und Polyamiden eingesetzt werden (Journal of Applied Polymer Science 1997, 65, 1813-1819; WO 0166633; WO 9634909).

Aus der Literatur sind dem Fachmann zahlreiche Synthesewege für die Herstellung von Phenylenbisoxazolinen bekannt.

Als besonders vorteilhaft hat sich die einstufige Synthese dieser Verbindungen ausgehend von Terephthalodinitril bzw. Isophthalodinitril und einem beliebig substituierten 1,2-Aminoalkohol erwiesen. Obwohl die Cyclisierung insbesondere bei aromatischen Nitrilen auch ohne Katalysator abläuft (DE 21 35 644), ist eine wirtschaftliche Synthese nur auf katalytischem Wege möglich.

Als Katalysatoren für die oben beschriebene Reaktion können beispielsweise gemäß DE 21 58 615, DE 21 27 776, CA-Nr. 94: 15 708 Salze anorganischer oder organischer Säuren von Metallen der 1., 2., 11. (IB) und 12. (IIB) Gruppe des Periodensystems dienen. Besonders häufig werden dabei die dem Fachmann bekannten Katalysatoren Zinkchlorid, Zinkacetat, Nickelacetat oder Cadmiumacetat eingesetzt. Darüber hinaus können laut US 3 741 961 auch z. B. Cobalt-, Chrom-, Eisen- und Kupferkatalysatoren für die gewünschte Heterocyclisierung verwendet werden.

Nachteil all dieser Verfahrensweisen ist zum einen der Einsatz von teils hochgiftigen Schwermetallverbindungen. Zum anderen sind die eingesetzten Schwermetallsalze nur zu einem geringen Teil im Reaktionsgemisch löslich und können insbesondere bei festen Phenylenbisoxazolin-Produkten nicht durch einfache Filtration vom Produkt abgetrennt werden, wie hier festgestellt wurde, im Gegensatz zu der Aussage aus DE 21 58 615 (Seite 5), nach der die Entfernung der Katalysatoren angeblich keine Schwierigkeiten bereiten würde. In den meisten Fällen ist somit zur Abtrennung des Katalysators durch Auflösen eine wässrige Aufarbeitung notwendig, bei der ein Schwermetall-belasteter Abwasserstrom anfällt, der kostenintensiv entsorgt werden muss.

Darüber hinaus weist vor allem das Verfahren nach DE 21 27 776 niedrige Ausbeuten von ca. 30 - 60 % und sehr lange Reaktionszeiten von bis zu 25 Stunden auf.

Jüngere Patentschriften beschreiben den Einsatz von Ru- oder Rh-Edelmetallkomplexen als geeignete Katalysatoren für die Oxazolinsynthese wie beispielsweise JP 06056803. Diese Verbindungen sind jedoch für eine großtechnische Produktion der Zielverbindungen zu teuer.

Dass zur Beschleunigung des Reaktionsumsatzes der jeweilige Aminoalkohol bevorzugt im Überschuss eingesetzt wird, ist Stand der Technik. Eine Recyclierung der eingesetzten Überschüsse wird in den oben genannten Literaturstellen jedoch nicht beschrieben. Im Falle einer wässrigen Aufarbeitung gelangt überschüssiger Aminoalkohol sogar zusammen mit dem Katalysator in den Abwasserstrom.

In der Literatur wird bezüglich der Edukte keine bevorzugte Dosierreihenfolge oder Dosierweise beschrieben. Es ist jedoch bekannt, dass es bei der Reaktion von Dinitril mit Aminoalkohol mit oder ohne Katalysator zur Bildung intensiv gefärbter organischer Nebenprodukte (Chromophore) kommt, was sich durch die weiter unten ausgeführte Nacharbeitung von DE 21 58 615 nochmals bestätigt hat. Dies führt insbesondere bei schwerlöslichen Produkten, wie dem 2,2'-(1,4-Phenylen)bis-[4,5-dihydro-1,3-oxazol], zu Problemen, da verfärbtes Produkt nicht durch konventionelle Methoden wie z. B. Umkristallisation gereinigt werden kann. Da sich solche Produktverfärbungen selbst wenn sie nur in Spuren auftreten in den nachfolgenden Produkten oder Mischungen, insbesondere bei Anwendungen in Polymeren, fortpflanzen und zu nachteiligen Effekten führen, sind solche Verfärbungen unerwünscht.

Es bestand demnach die Aufgabe, ein im technischen Maßstab durchführbares Verfahren bereitzustellen, das die oben genannten Probleme löst und es erlaubt, hochreine und insbesondere farblose Phenylenbisoxazoline unter Recyclierung eingesetzter Überschüsse an Reagenzien und gegebenenfalls verwendeter Lösungsmittel mit Hilfe verfügbarer, möglichst untoxischer Katalysatoren in möglichst hoher Raum-Zeitausbeute herzustellen.

Darüber hinaus sollte bei einem solchen Verfahren während der Aufarbeitung auf den Einsatz von Wasser oder wässrigen Mischungen verzichtet werden können und die Nachteile der herkömmlichen Verfahren vermieden werden.

Gelöst werden diese sowie weitere nicht explizit genannten Aufgaben, die jedoch aus den hierin diskutierten Zusammenhängen ohne weiteres ableitbar oder erschließbar sind durch ein Verfahren nach Anspruch 1. Zweckmäßige Ausformungen und Abwandlungen des erfindungsgemäßen Verfahrens werden in den auf Anspruch 1 rückbezogenen Unteransprüchen unter Schutz gestellt.

Dadurch dass man zur Herstellung von 1,3-Phenylenbisoxazolinen und 1,4-Phenylenbisoxazolinen der allgemeinen Formel I durch Umsetzung eines aromatischen Dinitrils der allgemeinen Formel II mit einem 2-Aminoalkohol der allgemeinen Formel III 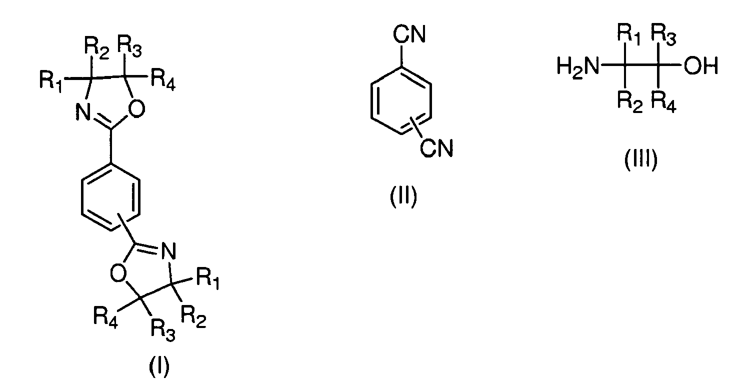 wobei R₁, R₂, R₃, R₄ gleich oder voneinander verschieden sein können und jeweils Wasserstoff, Methyl, Ethyl, n-Propyl oder iso-Propyl bedeuten können, in Gegenwart eines geeigneten Katalysators, ein Verfahren anwendet, bei dem der Katalysator, der Aminoalkohol und ggf. ein geeignetes Lösungsmittel bei Reaktionstemperatur vorgelegt werden und das jeweils eingesetzte Dinitril zudosiert wird, gelingt es in völlig überraschender Weise, die oben näher bezeichneten Nachteile des Standes der Technik zu überwinden.

Dabei ist es vorteilhaft, wenn das jeweils eingesetzte Dinitril als Feststoff, als Schmelze oder in einem geeigneten Lösungsmittel gelöst zudosiert wird.

Durch die erfindungsgemäße Verfahrensweise bleibt zudem die Konzentration an aromatischem Dinitril im Reaktionsgemisch während der gesamten Reaktion niedrig, was im Gegensatz zur an sich technisch einfacheren Zudosierung des Aminoalkohols zum vorgelegten Dinitril oder der gemeinsamen Vorlage der Edukte offenbar dazu beiträgt, dass deutlich weniger farbige Nebenprodukte gebildet werden. Erfindungsgemäß erhält man die hochreinen Produkte als farblose Kristalle, ohne dass ein zusätzlicher Reinigungsschritt notwendig wäre.

Als vorteilhaft hat sich dabei der Einsatz von Zinkverbindungen als Katalysator erwiesen, wobei sowohl eine Zinkverbindung als auch ein Gemisch von mehreren Zinkverbindungen nebeneinander eingesetzt werden können. Vorzugsweise werden dabei Zinkcarbonsäuresalze von gesättigten, aliphatischen Carbonsäuren mit zwei bis zehn Kohlenstoffatomen eingesetzt, die sowohl verzweigt als auch unverzweigt sein können, wie beispielsweise Zinkacetat, Zinkpropionat, Zink-n-butyrat, Zinkisobutyrat etc. oder auch von Zink-2-ethylhexanoat, die jeweils als Einzelkomponente oder als Gemisch von mehreren Komponenten eingesetzt werden können. Ganz besonders bevorzugt wird dabei jedoch die Verwendung von Zink-2-ethylhexanoat als Katalysator. Als 2-Aminoalkohol wird erfindungsgemäß bevorzugt 2-Aminoethanol eingesetzt.

Die vorzugsweise zu verwendenden Zinkcarbonsäuresalze bieten den Vorteil großer katalytischer Aktivität bei gleichzeitig geringstmöglicher Toxizität und einfacher Handhabbarkeit, bei gleichzeitig guter Verfügbarkeit.

Das molare Verhältnis von Aminoalkohol zu Dinitril beträgt dabei mindestens 2:1. Bevorzugt wird der Aminoalkohol jedoch in einem 4- bis 8-fachen molaren Verhältnis, bezogen auf das Dinitril eingesetzt. Dadurch wird die Konzentration an Dinitril während der Reaktion zusätzlich niedrig gehalten.

Die Reaktion wird erfindungsgemäß im Temperaturbereich von etwa 50 °C bis etwa 200 °C, vorzugsweise bei 100 °C bis 150 °C, durchgeführt, insbesondere aber bei einer Temperatur von 120 °C bis 140 °C.

Je nach Substrat und technischen Voraussetzungen wird die Reaktion vorzugsweise bei Drücken von etwa 0.5 bara (= bar absolut) bis 10 bara durchgeführt.

Dabei ist es insbesondere bei der Fahrweise unter leichtem Unterdruck (bezogen auf Normaldruck) von Vorteil, entstehendes Abgas damit schneller zu entfernen.

Die Reaktion wird jedoch ganz besonders bevorzugt bei Normaldruck durchgeführt, da hier der geringste Aufwand erforderlich ist.

Die Reaktion kann mit oder ohne Lösungsmittel durchgeführt werden. Geeignete Lösungsmittel sollten dabei unter den gewählten Reaktionsbedingungen inert sein und vorzugsweise mit dem eingesetzten Aminoalkohol ein Azeotrop bilden, welches sich bei Raumtemperatur wieder in seine Bestandteile auftrennt. Erfindungsgemäß bevorzugt werden dabei aromatische Kohlenwasserstoffe oder Etherverbindungen, die insbesondere bei Verwendung von 2-Aminoethanol die gewünschten Eigenschaften aufweisen.
Während der Synthese und der Isolierung der Phenylenbisoxazoline wird jedoch vorzugsweise kein Wasser zugesetzt. Generell ist es von Vorteil, den überschüssigen Aminoalkohol während und/oder im Anschluss an die Reaktion abzutrennen, vorzugsweise durch azeotrope Destillation mit einem geeigneten Lösungsmittel.

Dabei kann überschüssiger Aminoalkohol ohne wässrige Aufarbeitung durch azeotrope Destillation mit einem geeigneten Lösemittel wie z. B. Toluol, Xylol ― in reiner Form oder als Isomerengemisch ― oder Dibutylether im Falle von Ethanolamin in reiner Form isoliert und in die Reaktionsstufe zurückgeführt bzw. in weiteren Ansätzen wieder verwendet werden. Eine wässrige Aufarbeitung entfällt bei dem erfindungsgemäßen Verfahren somit völlig, was von großem Vorteil ist, weil damit gleichzeitig eine Entsorgung oder Aufbereitung belasteter Abwässer vermieden wird.

Die zu wählende Gesamtreaktionszeit, inklusive der Zeit der Zugabe von Reagenzien, ist dabei abhängig von der Temperatur und kann erfindungsgemäß von 1 bis 14 Stunden reichen. Bei Reaktionstemperaturen von 100 - 150 °C werden jedoch vorzugsweise Gesamtreaktionszeiten von 1 bis 4 Stunden, insbesondere von 1,5 bis etwa 3 Stunden angewendet. Diese relativ kurze Reaktionszeit bedeutet einen weiteren Vorteil gegenüber den bisher bekannten Verfahren, wie beispielsweise dem von DE 2158615 , bei dem deutlich längere Reaktionszeiten von 6 bzw. 12 Stunden gemäß Beispiel 1 bzw. 2 angewendet werden müssen.
Dadurch gelingt es die Raum-Zeit-Ausbeuten in vorteilhafter Weise zu erhöhen, was für die Wirtschaftlichkeit eines technischen Verfahrens eine ganz bedeutende Rolle spielt.

Die erfindungsgemäße Umsetzung von Dinitril mit Aminoalkohol kann sowohl diskontinuierlich, teilweise kontinuierlich oder aber auch kontinuierlich durchgeführt werden. Dies kann je nach technischen Voraussetzungen flexibel gehandhabt werden, was einen weiteren wichtigen Vorteil des Verfahrens darstellt.

Weiter kann es von Vorteil sein, am Ende der Reaktion vor dem Abkühlen der Reaktionsmischung eine geeignete Menge eines Lösungsvermittlers zuzusetzen, so dass die Reaktionsmischung bei Raumtemperatur eine homogene Flüssigphase bildet. Hier werden vorzugsweise kurzkettige lineare oder verzweigte aliphatische oder cycloaliphatische Alkohole mit einer Kettenlänge von C₁ bis C₆ verwendet, insbesondere jedoch Isopropanol. Auf diese Weise gelingt es kristallisierendes Produkt problemlos in einem Schritt z. B. durch Filtration von der Mutterlauge abzutrennen. Lösungsmittel und Lösungsvermittler können aus der Mutterlauge einfach destillativ zurückgewonnen werden und im Verfahren wieder eingesetzt werden.

Das erfindungsgemäße Verfahren führt außerdem zu Produkten mit hervorragender Reinheit von über 99,0 %, vorzugsweise sogar von bis zu über 99,5 %, die überraschenderweise auch keinerlei Verfärbungen mehr aufweisen, im Gegensatz zu Produkten aus herkömmlichen Verfahren.

Wie das nach DE 21 5 816 , Beispiel 1 ausgeführte Vergleichsbeispiel desweiteren zeigt, führt die dort angegebene Verfahrensweise zu einem deutlichen verfärbten Produkt im Gegensatz zum erfindungsgemäßen Verfahren.
Dies wird insbesondere am Vergleich der relativen Weißgrade, gemessen nach DIN 5033 (Prinzip 45/0), deutlich, bei dem das erfindungsgemäße Produkt mit Werten von 75 % bzw. 82 - 92 % deutlich besser abschneidet im Vergleich zum Produkt des Vergleichsbeispiels mit einem Wert von nur 39 % . Erfindungsgemäß ist es möglich, Weißgrade von > 70 %, bevorzugt von > 80 % insbesondere aber von > 90% zu erhalten.

Die erfindungsgemäßen Produkte können daher auch ohne weiteren Reinigungsaufwand direkt beispielsweise in Polymeren ― wie eingangs beschrieben ― weiterverwendet werden, was einen erheblichen Vorteil darstellt.

Darüber hinaus wurde nun überraschenderweise gefunden, dass flüssiges Zink-2-ethylhexanoat ganz generell hervorragend als Katalysator für die Synthese von Phenylenbisoxazolinen aus Terephthalodinitril oder Isophthalodinitril geeignet ist. Im Vergleich zu Zinkacetat wird die Reaktion mit Zink 2-ethylhexanoat deutlich beschleunigt. So zeigte etwa die Reaktion von 1 Molequivalent (moleq) Terephthalodinitril mit 2,28 moleq Ethanolamin und 0,05 moleq Zinkacetat-dihydrat auch nach über 7 h bei 132 °C immer noch erhebliche Mengen des als Zwischenprodukt entstehenden 4-(4,5-Dihydro-2-oxazolyl)-benzonitrils. Ein entsprechender Versuch mit 0,05 moleq Zink 2-ethylhexanoat zeigte dagegen bereits nach 5 h vollständigen Umsatz. Die Verwendung von Zink-2-ethylhexanoat ermöglicht damit eine deutliche Verbesserung der Raum-Zeit-Ausbeute im Vergleich zum herkömmlichen Einsatz von Zinkacetat oder anderer schwerer löslicher Zinksalze. Erfindungsgemäß werden bezogen auf eingesetztes Dinitril 0,1 - 5 Mol-%, vorzugsweise 1 - 2 Mol-% Zink-2-ethylhexanoat eingesetzt.
Ein weiterer Vorteil beim Einsatz von Zink-2-ethylhexanoat ist, dass dieser flüssige, im Reaktionssystem relativ gut lösliche Katalysator ohne wäßrige Aufarbeitung während der Filtration des Endproduktes in der Mutterlauge verbleibt und somit einfach und vollständig abgetrennt werden kann.

Zink-2-ethylhexanoat kann daher bei allen Verfahren zur Herstellung von 1,3-Phenylenbisoxazolinen und von 1,4-Phenylenbisoxazolinen der allgemeinen Formel I durch Umsetzung eines aromatischen Dinitrils der allgemeinen Formel II mit einem Aminoalkohol der allgemeinen Formel III vorteilhaft als Katalysator eingesetzt werden und löst insbesondere die Aufgaben einer verbesserten Raum-Zeit-Ausbeute, einer verbesserten Katalysatorabtrennung, der wasserfreien Aufarbeitung sowie der Durchführbarkeit im technischen Maßstab. Dem gemäß ist ein weiterer Aspekt der Erfindung ein Verfahren gemäß Anspruch 22.

Die folgenden Beispiele sollen das erfindungsgemäße Verfahren näher erläutern, nicht jedoch auf die speziell genannten Umstände einschränken.

Dabei wurden die folgenden analytischen Bestimmungsmethoden verwendet:
1. Produktreinheit per Gaschromatographie (GC):
   - Säule: fused silica, FS-SE-54
   - Mobile Phase: Stickstoff
   - Detektion: FID (Flammenionisationsdetektor)
   - Temperaturprogramm: Start: 50 °C, Rate: 10 °C/min, Ende: 250 °C
   - Injektionstemperatur: 250 °C
   - Flußrate: 2 ml/min
2. Produktreinheit per HPLC:
   - Säule: Hypersil MOS, 5 µm, 125 x 4 mm
   - Mobile Phase: Wasser, Methanol, es wird ein Gradient gefahren
   - Detektion: UV bei 242 bzw. 270 nm
   - Temperatur: Raumtemperatur
   - Flußrate: 1 ml/min
3. Weißgrad (R_{z}) in % durch Remissionsmessung am Feststoff nach DIN 5033 - insbesondere Teil 7 und Teil 9 - (Prinzip 45/0) und Vergleich mit einem Weißstandard.
   - Geräte: Colortester LFM1 (DR.LANGE), Emaille-Arbeitsstandard LZM 024 als unmittelbarem Weißstandard (gegen Referenzstandard Bariumsulfat = 100% Weißgrad geeicht), Pulverrundküvette mit 50 mm Durchmesser, Blaufilter (Z-Filter) mit Wellenlängenbereich 400 - 700 nm
   - Durchführung: Das Gerät wird zunächst mit dem Emaille-Arbeitsstandard geeicht. Anschließend wird die Probe in der Rundküvette mit ca. 2 cm Füllstand gleichmäßig eingefüllt und in die Meßvorrichtung des Colortesters eingebracht. Danach wird die Probe unter 45 ° im gewählten Wellenlängenbereich (400 - 700 nm) beleuchtet und die diffuse Reflexion des Lichts unter 0° gemessen und mit der des Weißstandards verglichen. Der R_{z}-Wert wird vom Gerät ausgegeben und gibt den relativen Weißgrad in % bezogen auf den Referenzstandard Bariumsulfat (= 100 %) an.

### Beispiel 1: Synthese von 2,2'-(1,3-Phenylen)bis-[4,5-dihydro-1,3-oxazol]

In einem 1 L-Mehrhalskolben mit Flügelrührer, Sumpfkontaktthermometer, Wasserabscheider, Rückflusskühler, Feststoffdosierer (mit Förderschnecke) und Stickstoffabdeckung wurden 244,3 g Ethanolamin (4 moleq), 318,5 g Xylol und 23,7 g Zink-2-ethylhexanoat bei Raumtemperatur vorgelegt.

Die zunächst 2-phasige Suspension wurde unter Rühren bis auf Rückflusstemperatur erhitzt. Anschließend wurden 128,1 g (1 moleq) Isophtalodinitril (IPN) über einen Zeitraum von 2Std. kontinuierlich als Feststoff zum heißen Sumpf zudosiert. Dabei entstand Ammoniak als Abgas.

Nach Ende der IPN-Dosierung wurde das nun einphasige Reaktionsgemisch ca. 1Std. bei Rückflusstemperatur nachgerührt.

Anschließend wurde überschüssiges Ethanolamin (EA) mit Hilfe eines Wasserabscheiders durch azeotrope Destillation soweit wie möglich aus dem Gemisch entfernt.

Das sich als separate Phase im Wasserabscheider sammelnde EA konnte direkt in weiteren Ansätzen eingesetzt werden.

Nach Abkühlen des Reaktionsgemisches auf 80 °C wurden 100 g i-PrOH zum Sumpf hinzugegeben. Anschließend wurde das homogene Gemisch unter Rühren weiter bis auf Raumtemperatur abgekühlt. Die ausgefallenen Kristalle wurden über eine Glasfilternutsche abgesaugt, zweimal mit Cyclohexan gewaschen und anschließend im Vakuum getrocknet.

Aus den organischen Wasch- und Filtrationslösungen konnten die verwendeten Lösungsmittel sehr einfach beispielsweise destillativ zurückgewonnen und in weiteren Ansätzen wieder eingesetzt werden.

Man erhielt 2,2'-(1,3-Phenylen)bis-[4,5-dihydro-1,3-oxazol] in Form farbloser, rieselfähiger Kristalle mit einer Reinheit >99,5 % (laut GC). Zusammen mit dem aus dem Filtrat durch Nachfällung isolierten Material lag die Gesamtausbeute an Zielprodukt bei 78 % d. Th.. Der Weißgrad (R_{z}) betrug 75 % (bezogen auf Bariumsulfat als Referenzstandard =100%) und nach Waschen mit Isopropanol 82%.

### Beispiel 2: 2,2'-(1,4-Phenylen)bis-[4,5-dihydro-1,3-oxazol]

In einem 1 L-Mehrhalskolben mit Flügelrührer, Sumpfkontaktthermometer, Rückflusskühler, Feststoffdosierapparatur (mit Förderschnecke) und Stickstoffabdeckung wurden 489 g Ethanolamin (8 moleq) bei Raumtemperatur mit 17,6 g Zink-2-ethylhexanoat (0,05 moleq) versetzt und unter Rühren auf eine Reaktionstemperatur von 130 °C erhitzt. Anschließend wurden 128 g Terephthalodinitril (= TPN, 1 moleq) innerhalb von 2Std.kontinuierlich als Feststoff in den beheizten Reaktionssumpf gefördert. Dabei entstand Ammoniak als Abgas.

Nach ca. 1Std. begannen aus der homogenen, schwach gelblichen Lösung farblose Kristalle auszufallen.
Anders als beim Vergleichsbeispiel kam es hier zu keiner Grün- oder Blaufärbung der Reaktionslösung. Nach Ende der TPN-Zugabe wurde das Reaktionsgemisch 1Std. bei 130 °C nachgerührt.

Das Reaktionsgemisch wurde auf Raumtemperatur abgekühlt und die ausgefallenen Kristalle wurden über eine Glasfilternutsche (Porengröße Nr. 4) abgesaugt, nacheinander zweimal mit je 90 g Methanol auf der Nutsche gewaschen und in einem Rotationsverdampfer bei 25-10 mbar und 80 °C Badtemperatur 4 Stunden lang getrocknet. Man erhielt 2,2'-(1,4-Phenylen)bis-[4,5-dihydro-1,3-oxazol] in Form farbloser, rieselfähiger Kristalle. Zusammen mit dem durch Nachfällung aus der Ethanolamin-Mutterlauge gewonnenen Produkt lag die Ausbeute der Reaktion bei 75 % d. Th. bei einer Reinheit des Produkts von > 99,5 Gew.% (laut HPLC). Der Weißgrad (R_{z}) betrug 92 % (bezogen auf Bariumsulfat als Referenzstandard =100 %).

Vergleichsbeispiel gemäß DE 2 158 615 Beispiel 1: 2,2'-(1,4-Phenylen) bis-[4,5-dihydro-1,3-oxazol] 92,3 g Terephthalsäuredinitril (TPN) wurden bei Raumtemperatur in 275 g Ethanolamin unter Rühren eingetragen. Dann wurden 1,45 g Zinkacetatdihydrat zugegeben und im Stickstoffstrom auf 100 °C erhitzt. Nach ca. 1 Std. war die Hauptmenge an TPN unter Abspaltung von Ammoniak in Lösung gegangen und die Lösung zeigte eine intensiv dunkelgrüne Färbung. Daraufhin wurden weitere 99,9 g TPN eingetragen, insgesamt also 1,5 Mol. Das Molverhältnis TPN:Ethanolamin betrug demnach 1:3. Die Katalysatormenge betrug 0,75 Gew.% bezogen auf TPN. Nach kurzer Zeit trat unter weiterer Ammoniakabspaltung Lösung ein. Bald darauf begann das Reaktionsprodukt aus der tiefgrünen Lösung auszufallen. Es wurde 6 Std. auf 105-110 °C erhitzt.
Danach lag das Reaktionsprodukt als dicker Brei im überschüssigen Ethanolamin vor. Während des Abkühlens verdünnte man durch Einrühren von 115 ml Isopropanol, wonach sich das Reaktionsprodukt leicht abfiltrieren ließ. Der Kristallbrei wurde auf dem Filter mit 95 g Isopropanol nachgewaschen. Danach wurde der Filterkuchen in viel Wasser (470 g) unter Rühren aufgeschlämmt, filtriert und mit Wasser (dreimal mit je 200 g) kräftig nachgewaschen, um das Ethanolamin quantitativ zu entfernen. Hiernach wurde noch mit Aceton (dreimal mit je 100 g) nachgewaschen, um die Hauptmenge Wasser zu entfernen, und anschließend getrocknet.
Es wurden 292 g grau-grünes Pulver enthalten, was einer Ausbeute von 89,9 % d. Th. an 2,2'-(1,4-Phenylen) bis-[4,5-dihydro-1,3-oxazol] entspricht, bezogen auf eingesetztes TPN.
Der Schmelzpunkt des Produktes lag bei 238 - 247 °C bei einer Reinheit von: 99,1 Gew.% (HPLC).
Der Weißgrad (R_{z}) betrug 39 % (bezogen auf Bariumsulfat als Referenzstandard =100%).

## Patentansprüche

1. Verfahren zur Herstellung von 1,3-Phenylenbisoxazolinen und von 1,4-Phenylenbisoxazolinen der allgemeinen Formel I durch Umsetzung eines aromatischen Dinitrils der allgemeinen Formel II mit einem Aminoalkohol der allgemeinen Formel III, 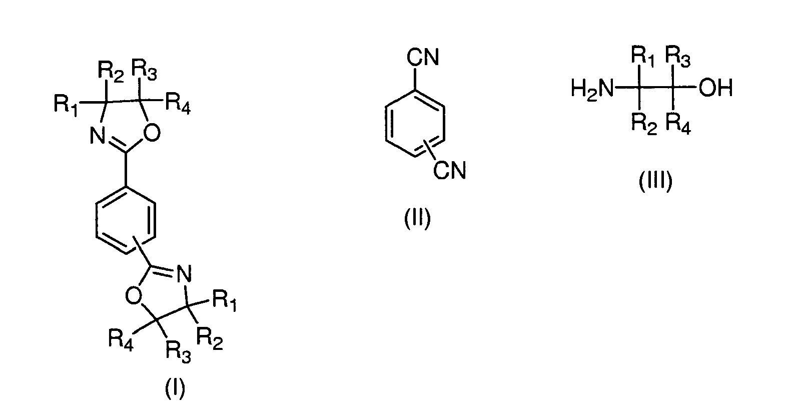 wobei R₁, R₂, R₃, R₄ gleich oder voneinander verschieden sein können und jeweils Wasserstoff, Methyl, Ethyl, n-Propyl oder iso-Propyl bedeuten können,
**dadurch gekennzeichnet,**
**dass** als Katalysator Zink-2-ethylhexanoat eingesetzt wird.

2. Verfahren nach Anspruch 1,
**dadurch gekennzeichnet,**
**dass** bezogen auf eingesetztes Dinitril 0,1 - 5 Mol-%, besonders bevorzugt 1 - 2 Mol-% Zink-2-ethylhexanoat als Katalysator eingesetzt werden.

## Claims

1. Process for preparing 1,3-phenylenebisoxazolines and 1,4-phenylenebisoxazolines of the general formula I by reacting an aromatic dinitrile of the general formula II with an amino alcohol of the general formula III 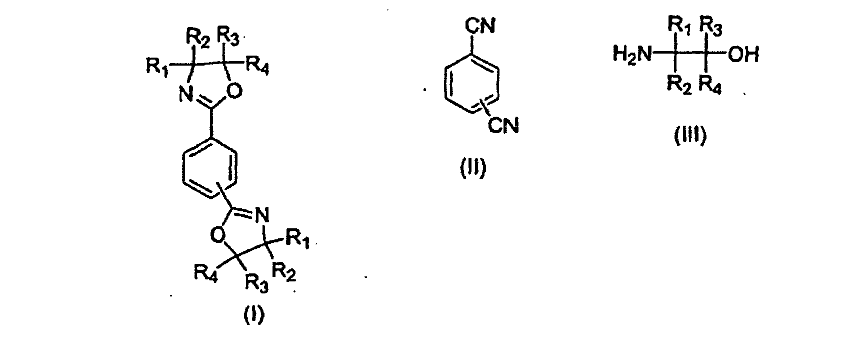 where R₁, R₂, R₃, R₄ can be identical or different and can each be hydrogen, methyl, ethyl, n-propyl or isopropyl,
**characterized in that** zinc 2-ethylhexanoate is used as catalyst.

2. Process according to Claim 1, **characterized in that** 0.1 - 5 mol%, particularly preferably 1 - 2 mol%, of zinc 2-ethylhexanoate, based on dinitrile used, is used as catalyst.

## Revendications

1. Procédé pour la préparation de 1,3-phénylènebisoxalines et de 1,4-phénylènebisoxalines de formule générale I, par mise en réaction d'un dinitrile aromatique de formule générale II avec un aminoalcool de formule générale III, 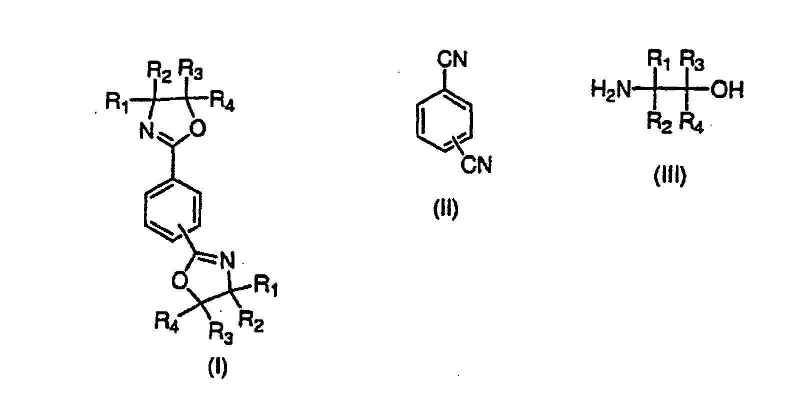 où R₁, R₂, R₃, R₄ peuvent être identiques ou différents et peuvent représenter chacun un atome d'hydrogène, un groupe méthyle, éthyle, n-propyle ou isopropyle,
**caractérisé en ce qu'**on utilise comme catalyseur du 2-éthylhexanoate de zinc.

2. Procédé selon la revendication 1, **caractérisé en ce qu'**on utilise comme catalyseur 0,1-5 % en moles, façon particulièrement préférée 1-2 % en moles de 2-éthylhexanoate de zinc par rapport au dinitrile utilisé.
